# EUROPEAN PATENT SPECIFICATION

(11) **EP 3 558 404 B1**
(45) Date of publication and mention of the grant of the patent: **07.06.2023**
(21) Application number: 17822278.2
(22) Date of filing: 20.12.2017
(51) Int. Cl.: A61L 27/12, A61L 27/56

(54) **BONE GRAFT SUBSTITUTE**
KNOCHENTRANSPLANTATERSATZ
SUBSTITUT DE GREFFE OSSEUSE

(30) Priority: 23.12.2016 EP 16206784
(43) Date of publication of application: 30.10.2019
(60) Divisional application: 22203780.6
(73) Proprietor: Collagen Matrix, Inc., Oakland, NJ 07436 (US)
(72) Inventor: PFISTER, Lukas, 8952 Schlieren (CH); RUFFIEUX, Kurt, 6003 Luzern (CH)
(74) Representative: Bohest AG Branch Ostschweiz
(86) International application number: PCT/EP2017/083829
(87) International publication number: WO 2018/115128

(56) References cited:
- US-A1- 2016 184 390
- US-B1- 6 302 913

## Description

### Field of invention

The present invention relates to a porous implant mass composition for treating defects in living organisms, such as bone defects and tooth extraction wounds and also relates to the manufacture thereof. More specifically, it relates to a new optimized geometry and pore structure allowing for a mechanically stable, but highly porous bone graft substitute.

### Background

Bone replacement materials are important in many fields such as, for instance, orthopaedics, dental surgery, traumatology and orthodontics. Such bone replacement materials are used, for example, to fill a hole left after removal of a bone tumour, to fill a bone defect after a fracture, to fill a void remaining after tooth extraction, to augment bone prior to dental or orthopaedic implant placement or to act as a carrier for antibiotics or osteogenic substances.

Bone replacement materials can be classified based on their origin, namely autograft materials, where the living organism's own bone is used, and bone graft substitutes, where the material comes from another source. Specific bone graft substitutes include allograft (human origin) materials, xenograft (animal origin) materials and synthetic materials.

Whilst autograft materials are osteoconductive as well as osteoinductive and have excellent healing potential, the amount that can be harvested is limited and donor site morbidity and associated pain are frequent for the patient.

For these reasons, bone graft substitutes are commonly used. Allograft and xenograft bone are osteoconductive and have a similar bone structure to the natural bone. However, there is a risk of disease transmission. Synthetic bone grafts can avoid the risk of disease transmission and are available in unlimited quantities. They also provide greater freedom to design and optimize the bone graft substitute for a good osteoconductivity.

For good osteoconduction, it is important that the graft material is in close contact with the bony walls. To achieve this, it is advantageous if the bone graft material can be formed into the required shape directly during surgery. For this reason, bone graft substitutes are most commonly provided as loose granules that can adapt to the contour of any defect.

Allografts and xenografts typically consist of trabecular bone chips, and many synthetic bone graft substitutes seek to mimic the natural trabecular structure of the bone chips because the trabecular structures provide a lot of space between the bone chips for bone in-growth. Unfortunately, these trabecular structures are very fragile and forming them into the required shape with sufficient pressure without breaking them is a major challenge.

In order to increase the stability of the granules, the walls can be compacted or the porosity within the granules can be reduced. However, both measures lead to reduced or no in-growth of the bone tissue. The use of spherical granules having a homogeneous internal porosity is found to increase resistance to breakage when placed into the defect. However, spherical granules do not provide much natural intergranular space that is desired for optimal bone ingrowth. If round spheres of identical size are densely arranged in a lattice packing, a porosity of only 26% can be calculated. By mixing small and large granules, this may even be reduced. Using spherical granules within the range of 500µm - 1000µm and which have the ability to be stuck together as described in patents WO-A-2005/107826 (Maspero et al), an intergranular space of 40 % is achieved.

In US 6'302'913 B 1 (Ripamonti et al), the inventors describe the benefit of implants with surface concavities accessible to the surrounding tissue. Their studies show fast bone formation within the cavities, which they name 'intrinsic osteoinductive activity'. They suggest that only offering a specific surface geometry (concavities of size 300 - 3000 µm) can trigger bone formation. However, all the studies were performed using discs, rods or modifying dental implant surfaces.

In US-2016/0184390, synthetic bone graft materials are described based on calcium ceramic granules, an osteoconductive protein and a biocompatible matrix. The calcium ceramic granules are disposed within the biocompatible matrix and have a specific surface area greater than about 30m²/g.

Thus, what is desired for optimal bone regeneration results is a granular material allowing contact to the surrounding bony walls when placed into the defect. The granules are preferably mechanically stable so that they do not break during insertion. The granules are preferably microporous allowing for fluid uptake. The mass preferably forms an interconnected highly porous scaffold with a large network of pores in between the granules. Ideally, the granules have surface concavities and are preferably linked to each other creating a mechanical stable mass.

### Summary of the invention

The present invention relates to a porous implant mass composition, providing an osteoconductive biocompatible bone graft substitute, for use in treating a defect in a living organism, comprising a plurality of biocompatible granules, as defined in claim 1 and to a method of manufacturing a porous implant mass composition for use in treating a defect in a living organism, comprising a plurality of biocompatible granules, wherein at least a portion of the granules have surface indentations, as defined in claim 13.

The implant according to the present invention overcomes the mentioned problems by providing an osteoconductive biocompatible bone graft substitute. The bone graft substitute combines substantially the high mechanical stability of spherical porous granules without the limitation of reduced intergranular space. The structure inside the granules has a high porosity whilst maintaining high stability, so that the granules can be pushed into a defect without risking significant breakage of the granules and, at the same time, the bone cells can grow into the space between the granules.

The surface of at least a portion of the granules comprises indentations, when viewed from the exterior of the granules. An indentation increases the porosity within the implanted mass significantly and thus provides more space between the granules for tissue ingrowth. Due to the indentations on the granules, the granules have an irregular shape and thus an increase in the intergranular space is achieved, while mechanical stability is maintained.

In one embodiment of the invention the granules are linked together. They are coated with a very thin layer of a biocompatible and resorbable thermoplastic polymer. The linking may be achieved by using a plasticizer such as a solvent or pressurized CO2 that lowers the glass transition temperature (Tg) of the coating. Subsequent condensing of the granular mass will result in pressing the coatings into each other or the coating of one granule into the mass of a second granule. Once the granules are pushed into a defect, the mass may be adapted to the geometry of the defect and thus adapted to the bony walls. Once the plasticizer is removed from the mass and the Tg rises again, a hardening of said mass occurs and thus a rigid implant forms.

In one embodiment of the invention the granules are linked together at a few contact points only to allow for a maximum of interconnected porosity within the mass. Such an implant composition according to the invention forms an open porous scaffold or composite matrix that allows in-growth and regeneration of bone tissue.

The granules have a specific surface area of less than 20m²/g, preferably less than 10m²/g, more preferably less than 5m²/g, and most preferably less than 3m²/g.

The granules can withstand a mean compressive force of at least 1N to ensure that the granules do not break during insertion.

In an exemplary embodiment, the moldable implant composition of the present invention includes a plurality of biocompatible granules mixed with a biocompatible polymer and a plasticizer for the polymer. The plasticizer is included in an amount sufficient to condition at least a portion of the biocompatible polymer such that the implant mass can be molded (i.e., is plastically deformable). The implant mass can be inserted in a bone defect where the implant mass can be deformed so as to assume the shape of the defect. The moldable implant composition can be deformed, molded, and/or sculpted to have any particular shape, either in-situ or ex-situ.

In one embodiment of the invention, the plasticizer is selected to cooperate with a hardening agent. Once the hardening agent is applied to the moldable implant composition, the effect of the plasticizer is neutralized and the implant composition hardens, thereby providing proper structural support. In an exemplary embodiment, the plasticizer is partially soluble in an aqueous solution such as a body fluid such that the body fluid can act as a hardening agent by extracting at least a portion of the plasticizer from the implant composition.

In one version of the invention, the softened implant composition is moldable, but is not so soft that it can flow like a liquid (i.e., it is not a fluid but it is plastically deformable). The advantage of a deformable implant is that its firmness allows the implant to maintain a desired shape until the hardener causes it to solidify.

In another embodiment of the invention, the implant composition is flowable and can take the shape of an implant site or a mold. This version of the invention can be advantageous where the desired shape of the implant is convoluted and/or difficult for a practitioner to form. By making the implant flowable, the implant mass can more easily take the shape of the implant site or the mold.

The moldable implant composition may also be shaped ex situ using a mold. The moldable implant composition of the present invention can easily deform to the shape of the mold and then be quickly hardened using a hardening agent. Shaping and hardening the implant composition in a mold according to methods of the present invention can save valuable time during a surgical operation thereby reducing costs and risks. For instance, after a tooth extraction a copy of the tooth root may be created using a mold ex situ. The implants of the present invention provide a practitioner with the ability to choose the best method for a particular situation.

In another embodiment of the present invention, the plurality of granules are formed from a bone tissue compatible ceramic made mostly of calcium phosphate or other calcium-based minerals. Implants made with calcium phosphate ceramics according to the present invention exhibit qualities such as the ability to (i) develop direct adhesion and bonding with existing bone tissue; (ii) promote cellular function and expression; (iii) provide a scaffold or template for the formation of new bone; and (iv) promote osteogenesis and act as a carrier for bioactive materials.

In another embodiment of the present invention, the plurality of granules are formed from synthetic biomaterial such as β-tricalcium phosphate, hydroxyapatite, mixtures thereof or other synthetically generated phases of calcium phosphate.

These and other features of the present invention will become more fully apparent from the following description and appended claims.

### Brief Description of the Drawings

To further clarify the above and other advantages and features of the present invention, a more particular description of the invention will be rendered by reference to specific embodiments thereof which are illustrated in the appended drawings. It is appreciated that these drawings depict only typical embodiments of the invention and are therefore not to be considered limiting of its scope.
- Fig. 1: illustrates typical spherical granules comprising calcium phosphate made without modification as described in the presented invention;
- Figs. 2a to 2c: illustrate exemplary implant compositions according to the invention having indentations of increasing size relative to the size of the granules; the granules being prepared using porogens within varying size ranges and at a constant pressure;
- Figs. 3a and 3b: illustrate further exemplary implant compositions according to the invention in which the granules are prepared using porogens within a single size range and a varying pressures;
- Figs. 4a to 4d: show samples made using cylindrical granules having indentations of increasing size relative to the size of the granules, wherein the granules are prepared using porogens within varying size ranges and at a constant pressure;
- Figs. 5a to 5c: illustrate the measurement of the intergranular space;
- Fig 6: shows the results of the intergranular porosity analysed of the spherical granules without indentation as well as the granules with exemplary indentations as described herein;
- Fig 7: illustrates the test method applied by which the force of failure of the granules is measured according to the present invention; and
- Fig. 8: shows the results for comparative force of failure testing of the granules according to the invention against commercially available granules.
- Fig. 9: shows that at least 75% of the pores between the sintered grains have a diameter of between about 1 and about 10 µm, as measured by mercury intrusion porosimetry.

### Detailed Description of the Drawings

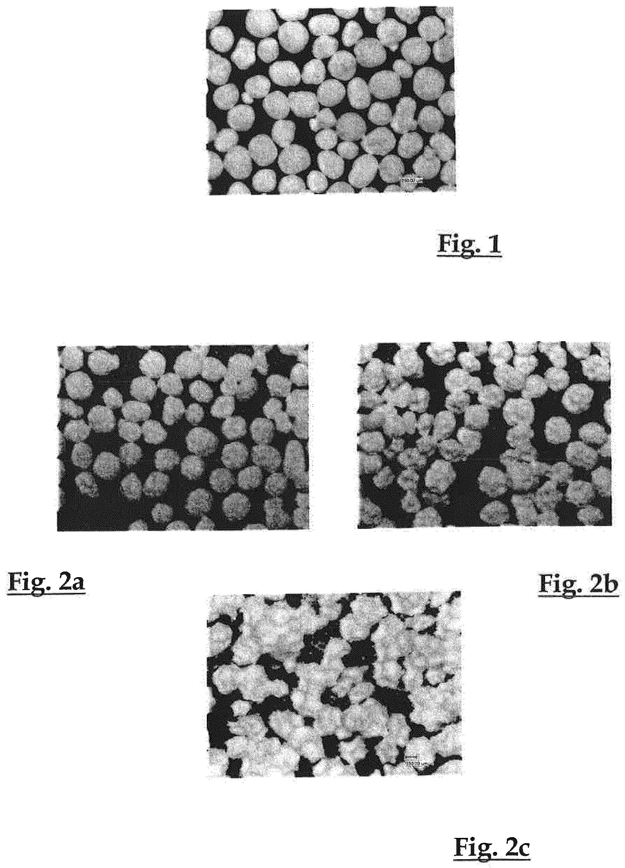

According to Figures 2a to 2c, granules according to Fig. 1 were modified by adding indentations from outside. The porogen used was cellulose spheres. The pressure applied was 100 MPa. In order to make these exemplary embodiments, different sizes of spherical porogen were used, namely 100 - 200 µm as shown in Figure 2a, 200 - 355 µm as shown in Figure 2b and 355 - 500 µm as shown in Figure 2c.

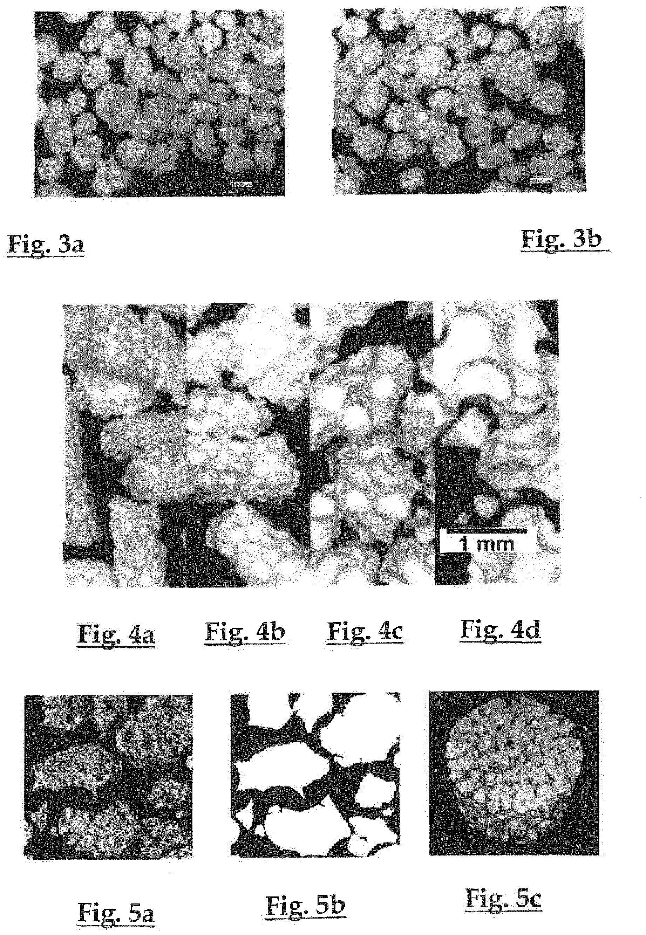

According to Figures 3a and 3b, granules according to Fig. 1 were modified by adding indentations from outside. The porogen used was cellulose spheres. The size of the porogen was selected to be within the range 355 - 500 µm. The pressure applied was either 10 MPa as shown in Figure 3a, or 26 MPa as shown in Figure 3b.

According to Figures 4a to 4d, the influence of different sized cellulose spheres on cyclindrical granules was demonstrated: from small indentation up to massive distortion of the cylindrical granules. The pressure applied was 100 MPa and the sizes of cellulose spheres used were selected to be in the range of from 100 - 200 µm as shown in Fig 4a, from 200 - 355 µm as shown in Fig 4b, from 355 - 500 µm as shown in Fig 4c and from 710 - 1000 µm as shown in Fig 4d.

According to Figures 5a to 5c, the intergranular space was measured by image analysis of 3-D reconstructed micro-CT slices through a defined cylindrical volume filled with granules. The porosity inside the granules was not measured, only the pores between the granules were analysed. a) micro-CT slice through the implant mass, b) removing the porosity within the granules by adjusting the contrast of the image, c) generating a 3-D model. Intergranular porosity is calculated volumetric using the 3-D model.

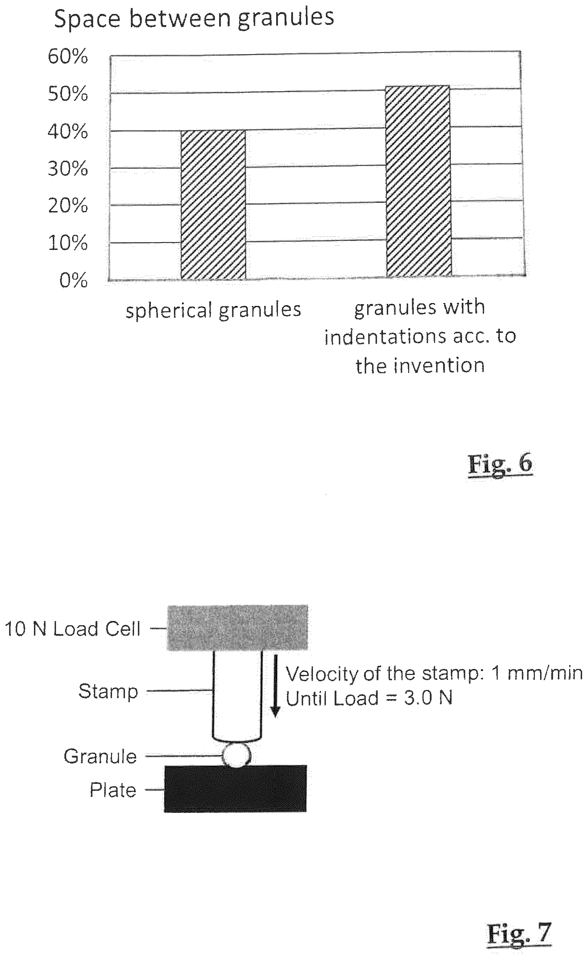

According to Figure 7, average size and average geometry single granules were chosen and placed in a mechanical test equipment. A 10N load cell was used and granules were loaded with a velocity of 1 mm/min until the granules broke.

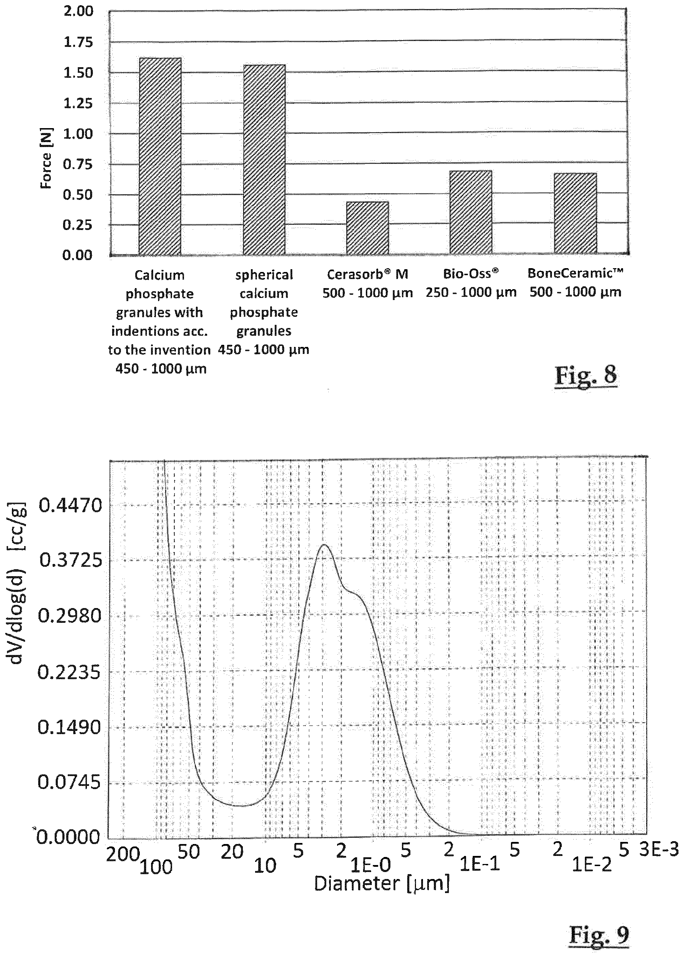

According to Figure 8, results show the significant higher mechanical force of failure of granules made according to the present invention as compared to highly porous competitor products (Bio-Oss^{®} (Geistlich, Switzerland), Cerasorb^{®}M (Curasan AG, Germany), BoneCeramic^{™} (Straumann, Switzerland)) with no reduction as compared to the spherical granules without indentations.

According to Figure 9, mercury intrusion porosimetry measurements were used to demonstrate that, in the granules according to the present invention, the pores between the sintered grains within the granules are homogenous. That is, at least 75% of the pores had an average diameter of between about 1 and about 10 µm.

### Description of Exemplary Embodiments

Embodiments of the present invention include moldable implant compositions for repairing a bone defect or wound. The moldable implant compositions are formed from a plurality of particle-like granules. A biocompatible polymer is disposed about or coated on the granules. The granules and polymer are packed or agglomerated to form an implant mass and the polymer is softened with a plasticizer to make the implant mass moldable or flowable. The implant mass is shaped or sculpted to form a bone implant that will fill a particular bone defect or structural void. The implant composition is then allowed or caused to harden. As discussed more fully below, the order and timing of (i) softening the polymer, (ii) forming the implant mass, and (iii) shaping the implant mass can vary according to different embodiments of the present invention.

So far, such implant compositions could only be made by using spherical granules, as a certain mechanical strength of the granules is needed to allow for (i) non-destructive coating and (ii) non-destructive insertion and condensation within the defect. However, this was limiting the porosity between the granules, reducing the amount of open space which is accessible by the tissue after implant mass insertion. According to the present invention, the inventors have found how intergranular porosity can be increased by adding surface concavities without reducing mechanical properties significantly.

### I. Components of the Implant Composition

The various components of an implant according the present invention will now be discussed.

In an exemplary embodiment, the present invention includes biocompatible granules, which are a hard substance that provides structural support or physiological advantages to the implant mass. The granules can be made of synthetic, naturally occurring, polymeric, or non-polymeric materials. In one embodiment, the granules are also degradable such that the implant degrades over time and/or be replaced with native bone tissue.

The biocompatible granules of the present invention can be made of a synthetic, biocompatible material, such as biopolymers, bioglasses, bioceramics, more preferably calcium sulphate, silicon oxide, calcium phosphate such as, for example, monocalcium phosphate monohydrate, monocalcium phosphate anhydrous, dicalcium phosphate dehydrate, dicalcium phosphate anhydrous, tetracalcium phosphate, calcium orthophosphate phosphate, calcium pyrophosphate, [alpha]-tricalcium phosphate, [beta]-tricalcium phosphate ([beta]-TCP), apatite such as hydroxyapatite (HA), or polymers such as, for example, poly([alpha]-hydroxyesters), poly(ortho esters), poly(ether esters), polyanhydrides, poly(phosphazenes), polypropylene fumarates), poly(ester amides), poly(ethylene fumates), poly(amino acids), polysaccharides, polypeptides, poly(hydroxy butytates), poly(hydroxy valerates), polyurethanes, poly(malic acid), polylactides, polyglycolides, polycaprolactones, poly(glycolide-co-trimethylene carbonates), polydioxanones, or copolymers, terpolymers thereof or blends of those: polymers, or a combination of biocompatible and degradable materials.

The biocompatible granules of the present invention can be made of naturally occurring materials such as xenografts or allografts or other animal or human derived hard substances. To those knowledgable in the art, it is clear that such materials can be ground to small particles and then granules may be made using known granulation and sintering techniques.

Calcium phosphate ceramics are biocompatible and can be used in various biomedical applications. Hydroxyapatite and β-TCP bioceramics and biphasic ceramics made thereof are particularly useful materials because they have similar ionic properties as the mineral components of bone. In addition, their resorption kinetics can be controlled to meet the needs of a specific therapy. Furthermore, because β-TCP is degradable, it is absorbed in vivo and can be replaced with newly formed bone.

The granules have an interconnected porosity within the granules which allows for body fluid to be taken up. Body fluids contain proteins and factors which support tissue regeneration. The use of porous granules reduces the amount of implanted materials and allows a better in situ integration.

The pores have an average homogeneous diameter of from about 1 µm to about 10 µm. By homogeneous it is meant that the granules have a homogeneous surface porosity defined by a diameter of at least 75% of the pores having a diameter of between about 1 and about 10 µm, as measured by mercury intrusion porosimetry.

In an exemplary embodiment, biocompatible granules are selected, which have a Feret-diameter of about 100 µm to about 4000 µm, and preferably from about 500 µm to about 1000 µm.

While the term Feret-diameter indicates that the granules may be of irregular shape, it can be advantageous to use granules of regular shape, such as spherical granules with indentations. In some applications, spherical granules allow a better handling and an easier estimation of the quantity required to fill a known volume of a cavity. Spherical or other regularly-shaped and/or sized granules form a more uniform pore structure or scaffold between the adjacent particles. However, draw-back of the well ordered structure is a low intergranular porosity. Thus it can be of advantage if the granules have an irregular shape. By irregular shape it is meant that substantially no granules are substantially spherical but have a shape in the form of e.g. rods, chips, tripods, or as described in more detail in the present invention, distorted granules.

Preferably the granules have an irregular surface. This simulates natural bone structure and helps the bone cells to grow into the matrix more effectively.

One example about how such granules can be made is described as follows. To form spherical granules of biphasic calcium phosphate with a ratio of 60 % hydroxyapatite (HA) and 40 % β-tri calcium phosphate (β-TCP), 155 grams of P-TCP and 232.5 g of HA powder of particle size of 1 µm to 30 µm were mixed with 112.5 g cellulose in a powder mixer. 250 millilitre of deionized water were slowly added to the powdery mixture under continuous stirring. The resultant mass was extruded through a multi-hole 800 µm-nozzle and spheronized for 3 min in a pellet-rounder to obtain granules having an average diameter of about 350 µm to about 1000 µm. The obtained granular green bodies were thereafter sintered at 1100 °C for 8 hours.

Other methods such as high-shear mixture and fluidized bed granulation can also be used to produce spherical granules. One example about how such granules can be made by fluidized bed granulation is described as follows. To form granules of β-TCP, 1.5 kilogram of β-TCP powder of particle size of 1 µm to 30 µm was placed together with 500 grams of cellulose in a fluidized bed granulator. The powder mixture was fluidized by an inlet air flow of 160 cubic meter per hour and a polyvinyl pyrrolidone solution was sprayed into the vessel to agglomerate the particles with a spray rate of 50 grams per minute. After 25 minutes, granules having an average diameter of about 350 µm to about 1000 µm were obtained. The obtained β-TCP granular green bodies were thereafter sintered at 1100 °C for 8 hours.

Within the scope of the invention, granules having a regular or irregular surface and shape and comprise indentations. Preferably the indentations are concavities or dimples. Indentations in the surface of the granule may be obtained by pressing a porogen into the surface of a granule whilst it is unhardened (in the example stated above this would mean before the sintering process). Depending on the shape of the porogen, different shaped indentations can be obtained. Preferably the porogen is spherical so as to provide concave indentations. Concave surface features preferably cover at least 25% of the surface of the granule. More preferably, concave surface features preferably cover at least 50% of the surface of the granule (see Figures 2 to 4).

According to a preferred embodiment, a method of manufacturing a porous implant composition for use in repairing a defect in a living organism, comprising a plurality of biocompatible granules wherein at least a portion of the granules having surface indentations, comprises
- manufacturing granules and mixing the granules with a porogen;
- pressing the porogen into the surface of at least a portion of the granules;
- removing the porogen from the implant mass so that indentations in the surface remain where the porogen was in contact with the granules.

An example of how such indentations in the surface of spherical granules can be generated is described as follows. Green body granules were used right after their manufacture. Cellulose spheres within the size range of 350 - 500 µm were mixed together with the still wet spherical green bodies in a Turbula mixer for 7 min at 25 rpm.

The so obtained mixture was filled into a cylindrical container and pressed in a hydraulic press at 16 - 24 MPa for 2 min. The cellulose spheres were thus pushed into the surface of the green body granules. The granules with indentations were then dried in a drying cabinet at 70 °C overnight. The obtained granular green bodies were thereafter sintered at 1100 - 1200 °C for 4 - 8 hours. After sintering, the granules were fractionated to obtain granules having an average diameter of from about 500 - 1000 µm.

A further example how such indentations in the surface of non-spherical granules in the form of sticks can be generated is described as follows. Green body granules as described above were used, although these were not rounded and dried in an oven. Cellulose spheres of various sizes from about 100 - 1000µm were mixed together with the green bodies. The so obtained mixture was filled into a cylindrical container and pressed twice in a hydraulic press at 100 MPa for 1 min. The so obtained sticks with indentations were then dried in a drying cabinet at 70 °C overnight. The obtained green bodies were thereafter sintered at 1100 - 1200 °C for 4 - 8 hours.

The porogen which may be used in the present invention can be any natural or synthetic substance which is removable from the implant composition by means of burning, melting, dissolving, leaching or mechanically removal. Residues of the porogen in the final product must be low enough to allow for biocompatibility of the implant composition. Examples of porogens include: polysaccharides and derivates therof, such as cellulose, microcrystalline cellulose, hydroxypropylcellulose, methylcellulose, hydroxypropyl methylcellulose, hydroxypropylmethylcellulose acetate succinate (AQOAT), ethylcellulose, carboxymethylethylcellulose, cellulose acetate phthalate, hydroxypropylmethylcellulose phthalate, croscarmellose; starch, processed starch; sodium starch glycolate, pregelatinized starch. Examples may also be synthetic polymers and copolymers made thereof, such as polymethylmethacrylate, polyethylene , polypropylene, polystyrene, polyvinylchloride, polyvinylpyrrolidone, polyvinyl alcohol, silicones, polylactides and polyglycolides. Further examples may also be salts, such as sodium chloride, potassium chloride, sodium bicarbonate. Further examples may also be ice or frozen substances.

The porogen can be used by itself or if desired, in a combination with two or more types of excipients. Preferably, salt may be removed by leaching, polymer and polysaccharides may be removed by burning, frozen substances may be removed by melting, and polymers may be removed by dissolving.

In order to analyse the increase of porosity in between the granules achieved by this new method, the intergranular porosity was measured by image analysis of 3-D reconstructed micro-CT slices through a defined cylindrical volume filled with granules (Figure. 5). Whereas intergranular porosity of the implant composition containing spherical granules was 40 %, adding indentations using the experiment described above led to a intergranular porosity of 51 %, which corresponds to an increase in intergranular porosity of 27.5 % (Figure. 6).

In order to measure the mechanical stability of the granules, average size and average geometry single granules were chosen and placed in a mechanical test equipment (Figure. 7). Force of mechanical failure of the granules according to the invention were compared against commercially available granules. At least 20 granules were tested per product. Results were as follows: the competitor products such as the xenogenic, bone trabecular shaped graft material Bio-Oss^{®} (Geistlich Switzerland), the synthetic, porous Cerasorb^{®} M (Curasan, Germany) as well as the synthetic bone trabecular shaped BoneCeramic^{™} (Straumann, Switzerland) showed force of failure in average far below 1 N. Granules having a spherical shape with the internal porosity concentrated in the core of the granule showed an average force of failure at 1.5 N. The method described in this invention achieved an increase in the intergranular porosity from 40 % to 51 % while not reducing the force at failure.

Results show the significant higher average mechanical force of failure of granules made according to the present invention as compared to highly porous competitor products and with no significant reduction as compared to the spherical granules without indentations.

The implant composition of the present invention may also include a biocompatible polymer disposed about the granules to form an implant mass. In one embodiment, a portion of or all of the granules are coated with the biocompatible polymer. In an exemplary embodiment, the biocompatible polymer is also degradable so as to promote absorption into the body as the implant is replaced by newly-formed living tissue.

Biocompatible and preferably resorbable polymers suitable for use in the present invention include poly([alpha]-hydroxyesters), poly(orthoesters), poly(ether esters), polyanhydrides, poly(phosphazenes), polypropylene fumarates), poly(ester amides), poly(ethylene fumarates), poly(amino acids), polysaccharides, polypeptides, poly(hydroxy butyrates), poly(hydroxy valerates), polyurethanes, poly(malic acid), polylactides, polyglycolides, polycaprolactones, poly(glycolide-co-trimethylene carbonates), polydioxanones, or copolymers, terpolymers thereof or blends of those polymers.

The synthetic biocompatible granules may be spray-coated, preferably in a fluidized bed machine, or immersion-coated with the desired biocompatible polymer(s). Both methods lead to the biocompatible granules having advantageous properties.

The spray coating process in a fluidized bed machine allows the fabrication of a great number of nearly identical polymer-coated biocompatible granules in a very fast and economic manner. Using the fluidized bed process allows control of the thickness of the coating layer(s) and the fabrication of biocompatible granules having multiple coating layers, which are distinct from each other. The coating in fluidized bed machine results in a homogenous and continuous coating. During the coating process the granules do not adhere to each other, thus avoiding the formation of undesirable aggregates which might lead to highly inhomogeneous size distributions and coating thickness.

Integration of additives such as plasticizers or biologically active substances into the coating(s) can be easily controlled by the fluidized bed machine. Thus, each granule is loaded with the same amount of the biologically active substances. The thickness of the coating is also easily controlled. Therefore, even the release of an integrated biologically active substance is predictable and well controlled.

The coating of the biocompatible granules may include one or more layers of varying average thickness. This embodiment of the invention allows providing biocompatible granules with several coatings for specific purposes. The outermost degradable coating may be selected in accordance with a certain desired delay in degradability. This, for example, allows a retarded delivery of a bioactive substance. Thus, the synthetic biocompatible granules may be coated with different coatings, each of which is degradable and displays a specific effect.

The biocompatible polymer coating preferably has a thickness of about 1 µm to about 300 µm, preferably of about 5 µm to about 30 µm. The coating thickness of the granules can also be expressed as a weight fraction of about 4 % to about 20 % coating materials of the total weight of the implant mass, which may be loaded with additives such as plasticizers or biologically active substances. Those skilled in the art will recognize that by selecting different coating solutions and varying the coating time, different layers of coatings having different thicknesses can be applied to granules.

The mechanical stability of an implant made of coated granules can depend on the thickness and the homogeneity of the coating. An insufficient coating thickness can cause the granules to fail to stick together. On the other hand, too much coating can cause a decrease in the pH in the vicinity of the implant during its degradation. Whether the thickness of the coating has an adverse effect on the performance of the implant depends on the particular use of the implant.

As explained before, it is beneficial to have a moldable mass in order to adapt the implant mass well to the defect. This can be achieved by adding a plasticizer to the biocompatible polymer which lowers the glass transition temperature (Tg) of the polymer. In one embodiment, the biocompatible polymer and the plasticizer are selected to work in a polymer-solvent system. The biocompatible polymer is selected to have a desired flexibility and tackiness when partially dissolved or softened in a particular plasticizer. When the plasticizer is removed (e.g., by evaporation or diffusion into the body), the biocompatible polymer hardens to form a rigid bone implant. The polymer and plasticizer are chosen to give the implant a particular stiffness when softened and hardened. The plasticizer can be a liquid or a gas such as pressurized CO2.

The plasticizer is preferably biocompatible or exhibits a very low toxicity such that it can safely exist in the bone implant once the implant has been placed in a patient. Suitable plasticizers include, but are not limited to, n-methyl-2-pyrrolidone (NMP), acetone, ethyl lactate, ethyl acetate, ethyl formiate, acetyltributyl citrate, triethyl citrate, tetrahydrofuran, toluene, alcohol and carbon dioxide. Those skilled in the art will recognize that the plasticizer of the present invention can be one of many other solvents or a combination of solvents that condition the biocompatible polymers of the present invention.

In an exemplary embodiment, the plasticizer is a solvent that has solubility in aqueous medium, ranging from miscible to dispersible. Thus, the plasticizer is capable of diffusing into an aqueous medium or into body fluids such as, for example, tissue fluids, such as blood serum, lymph, cerebral spinal fluid, and saliva. When the plasticizer diffuses out of the implant mass, the bone implant is caused to harden. In this way, body fluids can be used as a hardener to solidify the bone implant in-situ.

The bone implant can also be hardened ex-situ by drawing the plasticizer out of the polymer. In one embodiment, the plasticizer is selected to be partially soluble in water. Once the implant is shaped ex-situ, such as in a mold, water is placed on the implant, thereby extracting the plasticizer and hardening the bone implant. Alternatively, the plasticizer can be removed by evaporation (e.g., by heating and/or applying a vacuum).

The solubility or miscibility of the degradable polymer in a particular plasticizer may vary according to factors such as crystallinity, hydrophilicity, capacity for hydrogen bonding, and molecular weight. Consequently, the molecular weight and concentration of the biocompatible polymer can be adjusted to modify the plasticizer's solubility. As mentioned above, to form a moldable implant, the polymer-plasticizer system is designed such that the plasticizer softens the polymer but does not liquefy the polymer, thereby creating a sticky, pliable mass.

In one embodiment, the polymer-solvent system is designed to reduce the Tg of the biocompatible polymer to a temperature below room temperature. For example, acetone, NMP, or an alcohol is added to poly-lactic-co-glycolic acid (PLGA) until the Tg of the PLGA drops from about 45-55 °C to below room temperature. Likewise, other polylactides and copolymers thereof having a Tg in the range of 40 - 65 °C can be lowered to below room temperature with the plasticizer.

In another embodiment, the bone implant can be shaped and/or molded without plasticizer provided the preparation is carried out above Tg. Thus, in this embodiment, the implant can be heated above Tg to make the implant moldable for implantation in a person or a mold without any plasticizer. The bone implant hardens as the temperature decreases if it has been shaped at a temperature higher than 37 °C. Prerequisite is that the Tg of the polymer is higher than the body temperature (37 °C.), which is the case for several polymers mentioned above.

According to one embodiment of the present invention, the bone implant has macro-pores and/or micro-pores that form an open porous scaffold or composite matrix. The term "open porous scaffold" or "composite matrix" refers to a structural matrix of granules that are bonded or otherwise joined together so as to define a granular region comprising solid or porous granules and an open porous region comprising spaces or discontinuities between adjacent granules of the granular region. The open porous region may be filled with air or gas at least initially, or it may be at least partially filled with liquid, solid particles, gel, and the like.

The scaffold or composite matrix can be obtained by fusing together granular biomaterial such as polymeric granules and/or coated granules. The scaffold or composite matrix of the biocompatible implant may be made of granules with indentations having micropores with average diameters of about larger than 0 to about 10 µm. By the fusion of the granules, the microporosity remains and/or macropores between the granules are formed having average diameters of about more than 10 µm to about 2000 µm, preferably about 100 µm to about 500 µm.

It can be advantageous in some cases to provide a biocompatible scaffold or composite matrix, which includes both coated and non-coated granules. The coated and uncoated granules can be thoroughly mixed such that they fuse together and still have the needed stability. By providing a mixture of coated and non-coated granules for the production of the biocompatible implants, the amount of coating materials, which must degrade, may be further reduced.

The bone implant can also include a membrane on an outer surface, which prevents soft tissue in-growth and/or contamination and leaves space open for regeneration away from the outer surface. The biocompatible membrane can be a degradable polymer film, polymer textile, polymer fleece or layer of interconnected fused polymer particles or a combination thereof and sealed to the implant, thus forming at least one layer of impermeability to soft tissue and epithelial cells.

In an embodiment of the invention, the membrane is made of a synthetic, biocompatible and degradable polymer selected from the group including poly([alpha]-hydroxyesters), poly(ortho esters), poly(ether esters), polyanhydrides, poly(phosphazenes), polypropylene fumarates), poly(ester amides), poly(ethylene fumarates), poly(amino acids), polysaccharides, polypeptides, poly(hydroxy butyrates), poly(hydroxy valerates), polyurethanes, poly(malic acid), polylactides, polyglycolides, polycaprolactones, poly(glycolide-co-trimethylene carbonates), polydioxanones, or copolymers, terpolymers thereof or blends of those polymers.

Cell occlusive properties can also be achieved by fusing granules or coated granules together. If much force is applied, the coating or the linking polymer particles may fill up all intergranular porosity. Granules used for this purpose preferably have a size smaller than about 500 µm and more preferably between about 1 µm to 200 µm.

### II. Formation of Implant Composition

As mentioned above, formation of the implant composition includes (i) softening the polymers as to form an implant mass that is moldable (i.e., plastically deformable); and (ii) shaping the moldable implant mass into a desired shape (ex situ or in situ). In various embodiments of the present invention, these steps are performed in a different order and/or simultaneously. Unless otherwise specified, the term "unshaped" means an implant mass that needs a substantial amount of molding to reach its final shape in a patient. The term "shaped" means an implant that is sufficiently shaped such that it needs little or no molding to function as an implant in a patient.

For instance, an unshaped implant mass is formed and then softened. Coated granules are packed to form an unshaped implant mass. Implant mass has little or no plasticizer such that it is hard. Unshaped implant mass can be easily stored or shipped without affecting the implant's condition.

Alternatively, implant mass is submerged in a liquid plasticizer. The biocompatible polymer of implant mass and the plasticizer are selected such that the biocompatible polymer absorbs the plasticizer. Unshaped implant mass is left in the plasticizer until implant mass absorbs enough plasticizer to be sufficiently moldable, but not completely dissolved or softened so much as to yield a soapy liquid that is not moldable.

In an alternative embodiment, an initially hard and unshaped implant mass is conditioned using a plasticizer to yield a softened (or moldable) implant mass. Moldable implant mass is then forced into a mold to form a shaped implant mass. The mold can have any desired mold cavity (e.g. the shape of an extracted tooth root, a cylinder, or other regular or irregular shape).

A hardener is added to shaped implant mass in the mold, e.g. using a syringe. The hardener is a liquid selected to extract or neutralize the plasticizer. In one embodiment, the hardener is a substance in which plasticizer is soluble. Thus, the hardener draws the plasticizer out of the shaped implant mass thereby forming a hardened implant composition. In an exemplary embodiment, the hardener is water. Finally, the hardened implant mass is extracted from the mold.

Thus, in accordance with the invention there is described a bone graft substitute which combines substantially the high mechanical stability of spherical porous granules without the limitation of reduced intergranular space. The structure inside the granules has a high porosity whilst maintaining high stability, so that the granules can be pushed into a defect without risking significant breakage of the granules and, at the same time, the bone cells can grow into the space between the granules. The surface of at least a portion of the granules comprises indentations, when viewed from the exterior of the granules. An indentation increases the porosity within the implanted mass significantly and thus provides more space between the granules for tissue ingrowth. Due to the indentations on the granules, the granules have an irregular shape and thus an increase in the intergranular space is achieved, while mechanical stability is maintained.

The invention has been described hereinbefore with reference to various embodiments. The description of these concrete embodiments only serves for explanation and a deeper understanding of the invention and is not to be considered as limiting the scope of the invention. Rather, the invention is defined by the annexed claims and the equivalents that are apparent to the one skilled in the art and which are in accordance with the general inventive concept.

## Claims

1. A porous implant mass composition, providing an osteoconductive biocompatible bone graft substitute, for use in treating a defect in a living organism, comprising a plurality of biocompatible granules,
at least a portion of the granules having surface indentations;
wherein the individual granules have a specific surface area of less than 20 m²/g, and contain a network of interconnected pores,
the interconnected pores within the granules having a homogeneous surface porosity defined by a diameter of at least 75% of the pores being between about 1 µm and about 10 µm between sintered grains as measured by mercury intrusion porosimetry,
wherein the single granules can withstand a mean compressive force of at least 1N, as measured with a universal testing machine having a load cell, wherein at least 20 granules are loaded with a velocity of 1 mm/min until they brake, so that the granules can be pushed into a defect without risking significant breakage of the granules.

2. An implant composition as defined in claim 1, wherein the implant mass is synthetic.

3. An implant composition as defined in any one of the preceding claims, the biocompatible granules comprising a material selected from the group consisting of biocompatible ceramics, biocompatible glasses, and combinations thereof.

4. An implant composition as defined in any one of the preceding claims, the biocompatible granules comprising a material selected from the group consisting of silicon oxide, calcium sulphate, calcium phosphate, and combinations thereof.

5. An implant composition as defined in claim 4, the biocompatible granules comprising a material selected from the group consisting of monocalcium phosphate monohydrate, monocalcium phosphate anhydrous, dicalcium phosphate dehydrate, dicalcium phosphate anhydrous, tetracalcium phosphate, calcium orthophosphate, calcium pyrophosphate, α-tricalcium phosphate, β-tricalcium phosphate, hydroxyapatite, carbonate hydroxyapatite, apatite, bioglass, and combinations thereof.

6. An implant composition as defined in any one of the preceding claims, wherein the network of interconnected pores allows the integration of cells.

7. An implant composition as defined in any one of the preceding claims, wherein the granules have surface indentations, preferably concavities.

8. An implant composition as defined in any one of the preceding claims, wherein the granules are irregularly-shaped.

9. An implant composition as defined in any one of the preceding claims, wherein the granules have an irregular surface.

10. An implant composition as defined in any one of the preceding claims, wherein the granules have a Feret diameter of 100 µm to about 4000 µm.

11. An implant composition as defined in any one of the preceding claims, wherein the granules have a Feret diameter of 500 µm to about 1500 µm.

12. An implant composition as defined in any one of the preceding claims, wherein at least a portion of granules are coated with a biocompatible polymer and comprises a plasticizer in the implant composition in an amount sufficient to condition at least a portion of the biocompatible polymer so that the implant composition is plastically deformable into a desired shape and then hardenable upon removal of at least a portion of the plasticizer from the implant composition.

13. A method of manufacturing a porous implant mass composition for use in treating a defect in a living organism, comprising a plurality of biocompatible granules wherein at least a portion of the granules have surface indentations, comprising:
manufacturing granules and mixing the granules with a porogen;
pressing the porogen into the surface of at least a portion of the granules;
removing the porogen from the implant mass so that indentations in surface are obtained where the porogen is pressed into the granules.

14. A method as defined in claim 13, wherein at least a portion of the granules are in spherical form.

15. A method as defined in claim 13 or claim 14, wherein the porogen is in the form of particles, and at least a portion of the surface of the particles have a convex shape.

16. A method according to any one of claims 13 to 15 wherein the porogen is in the form of particles, and at least a portion of the particles are spherical.

17. A method as defined in any one of claims 13 to 16, wherein at least a portion of the porogen has a diameter in the range of 10 to 250 % of the diameter of the granules.

18. A method as defined in any one of claims 13 to 17, wherein the weight of porogen used is in the range of 20% to 80 % by weight of the amount of granules.

19. A method as defined in any one of claims 13 to 18, wherein the porogen comprises at least one member selected from the group consisting of ice, salt, polyethylene, silicon, polystyrene and cellulose.

20. A method as defined in any one of claims 13 to 19, wherein the porogen comprises cellulose.

21. A method as defined in any one of claims 13 to 20, wherein the step of removing the porogen comprises burning out, sieving, dissolving, melting or vaporizing.

22. A method as defined in any one of claims 13 to 21, wherein the step of removing the porogen comprises burning out the porogen.

## Patentansprüche

1. Poröse Implantatmassenzusammensetzung, die ein osteokonduktives biokompatibles Knochentransplantatersatzmaterial zur Verwendung bei der Behandlung eines Defekts in einem lebenden Organismus bereitstellt, umfassend
eine Vielzahl von biokompatiblen Körnchen, wobei mindestens ein Teil der Körnchen Oberflächenvertiefungen aufweist;
wobei die einzelnen Körnchen eine spezifische Oberfläche von weniger als 20 m2/g aufweisen und ein Netzwerk von miteinander verbundenen Poren enthalten,
wobei die miteinander verbundenen Poren innerhalb der Körnchen eine homogene Oberflächenporosität aufweisen, definiert durch einen Durchmesser der Poren zwischen den gesinterten Körnern zwischen etwa 1 µm und etwa 10 µm von mindestens 75 % der Poren, gemessen durch Quecksilber-Intrusionsporosimetrie, wobei die einzelnen Körnchen einer mittleren Druckkraft von mindestens 1 N standhalten können, sodass die Körnchen in den Defekt gedrückt werden können, ohne das Risiko nennenswerter Brüche, wobei die mittlere Druckkraft mit einer Universalprüfmaschine mit einer Kraftmessdose an mindestens 20 Körnchen gemessen wird, wobei diese Körnchen mit einer Geschwindigkeit von 1 mm/min belastet werden, bis sie brechen.

2. Die Implantatzusammensetzung gemäss Anspruch 1, wobei die Implantatmasse synthetisch ist.

3. Die Implantatzusammensetzung gemäss irgendeinem der vorangehenden Ansprüche, wobei die biokompatiblen Körnchen ein Material aus der Gruppe bestehend aus biokompatiblen Keramiken, biokompatiblen Gläsern und Kombinationen davon, aufweisen.

4. Die Implantatzusammensetzung gemäss irgendeinem der vorangehenden Ansprüche, wobei die biokompatiblen Körnchen ein Material aus der Gruppe bestehend aus Silikonoxid, Kalziumsulfat, Kalziumphosphat und Kombinationen davon, aufweisen.

5. Die Implantatzusammensetzung gemäss Anspruch 4, wobei die biokompatiblen Körnchen ein Material aus der Gruppe bestehend aus Monocalciumphosphat-Monohydrat, anhydrisches Monocalciumphosphat, Dicalciumphosphat-Dehydrat, anhydrisches Dicalciumphosphat, Tetracalciumphosphat, Calciumorthophosphat, Calciumpyrophosphat, α-Tricalciumphosphat, β-Tricalciumphosphat, Hydroxyapatit, Carbonat-Hydroxyapatit, Apatit, Bioglas und Kombinationen davon, aufweisen.

6. Die Implantatzusammensetzung gemäss irgendeinem der vorangehenden Ansprüche, wobei das Netzwerk der miteinander verbundenen Poren die Integration von Zellen ermöglicht.

7. Die Implantatzusammensetzung gemäss irgendeinem der vorangehenden Ansprüche, wobei die Körnchen Vertiefungen an der Oberfläche aufweisen, vorzugsweise Konkavitäten.

8. Die Implantatzusammensetzung gemäss irgendeinem der vorangehenden Ansprüche, wobei die Körnchen unregelmässig geformt sind.

9. Die Implantatzusammensetzung gemäss irgendeinem der vorangehenden Ansprüche, wobei die Körnchen eine unregelmässige Oberfläche aufweisen.

10. Die Implantatzusammensetzung gemäss irgendeinem der vorangehenden Ansprüche, wobei die Körnchen einen Feret-Durchmesser zwischen 100 µm und ungefähr 4000 µm haben.

11. Die Implantatzusammensetzung gemäss irgendeinem der vorangehenden Ansprüche, wobei die Körnchen einen Feret-Durchmesser zwischen 500 µm und ungefähr 1500 µm haben.

12. Die Implantatzusammensetzung gemäss irgendeinem der vorangehenden Ansprüche, wobei zumindest ein Teil der Körnchen beschichtet ist mit einem biokompatiblen Polymer, wobei die Implantatzusammensetzung einen Weichmacher enthält in einer Menge, die es ermöglicht, zumindest einen Teil des biokompatiblen Polymers derart zu konditionieren, dass die Implantatzusammensetzung zuerst plastisch in eine gewünschte Form bringbar ist und dann, nach Entfernung zumindest eines Teils des Weichmachers aus der Implantatzusammensetzung, härtbar.

13. Verfahren zum Herstellen einer porösen Implantatmassenzusammensetzung zur Verwendung bei der Behandlung eines Defekts in einem lebenden Organismus, wobei die Implantatmassenzusammensetzung eine Vielzahl an biokompatiblen Körnchen umfasst, wobei zumindest ein Teil der Körnchen Oberflächenvertiefungen aufweist, das Verfahren umfasst die Schritte:
Herstellen von Körnchen und Mischen der Körnchen mit einem Porogen
Pressen des Porogens in die Oberfläche von zumindest einem Teil der Körnchen
Entfernen des Porogens aus der Implantatmasse , sodass an Stellen, an denen das Porogen in die Oberfläche der Körner gepresst wurde, Vertiefungen erhalten werden.

14. Das Verfahren nach Anspruch 13, wobei zumindest ein Teil der Körnchen eine sphärische Form haben.

15. Das Verfahren nach Anspruch 13 oder 14, wobei das Porogen in Form von Partikeln vorliegt und mindestens ein Teil der Oberfläche der Partikel eine konvexe Form aufweist.

16. Das Verfahren nach irgendeinem der Ansprüche 13 bis 15, wobei das Porogen in Form von Partikeln vorliegt und mindestens ein Teil der Partikel sphärisch ist.

17. Das Verfahren nach irgendeinem der Ansprüche 13 bis 16, wobei zumindest ein Teil des Porogens einen Durchmesser hat, der 10% bis 250% des Durchmessers der Körnchen beträgt.

18. Das Verfahren nach irgendeinem der Ansprüche 13 bis 17, wobei das Gewicht des eingebrachten Porogens zwischen 20% und 80% des Gewichtes der eingebrachten Körnchen ist.

19. Das Verfahren nach irgendeinem der Ansprüche 13 bis 18, wobei das Porogen zumindest einen Bestandteil, ausgewählt aus der Gruppe bestehend aus Eis, Salz, Polyethylen, Silizium, Polystyrol und Zellulose, aufweist.

20. Das Verfahren nach irgendeinem der Ansprüche 13 bis 19, wobei das Porogen Zellulose aufweist.

21. Das Verfahren nach irgendeinem der Ansprüche 13 bis 20, wobei der Schritt des Entfernens des Porogens Ausbrennen, Sieben, Auflösen, Schmelzen oder Verdampfen aufweist.

22. Das Verfahren nach irgendeinem der Ansprüche 13 bis 21, wobei der Schritt des Entfernens des Porogens das Ausbrennen des Porogens umfasst.

## Revendications

1. Composition de masse d'implant poreuse, fournissant un substitut de greffe osseuse biocompatible et ostéoconducteur, pour utilisation dans le traitement d'un défaut chez un organisme vivant, comprenant de multiples granules biocompatibles,
- au moins une partie desquels granules présentent des indentations de surface,
- lesquels granules présentent individuellement une aire spécifique de moins de 20 m²/g et renferment un réseau de pores interconnectés,
- lesquels pores interconnectés au sein des granules présentent une porosité de surface homogène, définie par le fait qu'un diamètre d'au moins 75 % des pores entre grains frittés, mesuré par porosi-métrie par intrusion de mercure, vaut entre environ 1 µm et environ 10 µm,
- et dans laquelle les granules individuels peuvent résister à une force moyenne de compression d'au moins 1 N, force mesurée à l'aide d'une machine d'essais universelle comportant une cellule de charge dans laquelle au moins 20 granules sont chargés avec une vitesse de 1 mm/min jusqu'à ce qu'ils se rompent, de sorte que les granules peuvent être tassés dans un défaut sans risque significatif de rupture des granules.

2. Composition d'implant conforme à la revendication 1, dans laquelle la masse d'implant est synthétique.

3. Composition d'implant conforme à l'une des revendications précédentes, dans laquelle les granules biocompatibles comprennent un matériau choisi dans l'ensemble constitué par les céramiques bio-compatibles, les verres biocompatibles et les combinaisons de ces matériaux.

4. Composition d'implant conforme à l'une des revendications précédentes, dans laquelle les granules biocompatibles comprennent un matériau choisi dans l'ensemble formé par les oxyde de silicium, sulfate de calcium, phosphate de calcium et leurs combinaisons.

5. Composition d'implant conforme à la revendication 4, dans laquelle les granules biocompatibles comprennent un matériau choisi dans l'ensemble constitué par les suivants : phosphate monocalcique monohydraté, phosphate monocalcique anhydre, phosphate dicalcique dihydraté, phosphate dicalcique anhydre, phosphate tétracalcique, orthophosphate de calcium, pyrophosphate de calcium, phosphate tri-calcique alpha, phosphate tricalcique bêta, hydroxyapatite, hydroxy-apatite carbonatée, apatite, bioverre, et leurs combinaisons.

6. Composition d'implant conforme à l'une des revendications précédentes, dans laquelle le réseau de pores interconnectés permet l'intégration de cellules.

7. Composition d'implant conforme à l'une des revendications précédentes, dans laquelle les granules présentent des indentations de surface, de préférence des concavités.

8. Composition d'implant conforme à l'une des revendications précédentes, dans laquelle les granules sont de forme irrégulière.

9. Composition d'implant conforme à l'une des revendications précédentes, dans laquelle les granules ont une surface irrégulière.

10. Composition d'implant conforme à l'une des revendications précédentes, dans laquelle les granules présentent un diamètre de Féret valant de 100 µm à environ 4000 µm.

11. Composition d'implant conforme à l'une des revendications précédentes, dans laquelle les granules présentent un diamètre de Féret valant de 500 µm à environ 1500 µm.

12. Composition d'implant conforme à l'une des revendications précédentes, dans laquelle au moins une partie des granules sont revêtus d'un polymère biocompatible et qui comprend un plastifiant, présent dans la composition d'implant en une quantité suffisante pour mettre au moins une partie du polymère biocompatible dans un état tel que la composition d'implant peut subir une déformation plastique pour prendre une forme voulue et être ensuite durcie par élimination d'au moins une partie du plastifiant de la composition d'implant.

13. Procédé de fabrication d'une composition de masse d'implant poreuse, pour utilisation dans le traitement d'un défaut chez un organisme vivant, comprenant de multiples granules biocompatibles et dans laquelle au moins une partie de ces granules présentent des indentations de surface, comprenant les étapes suivantes :
- fabriquer des granules et mélanger ces granules avec un agent porogène,
- faire pénétrer par pression l'agent porogène dans la surface d'au moins une partie des granules,
- et éliminer l'agent porogène de la masse d'implant, de manière à obtenir des indentations de surface là où l'on a fait pénétrer l'agent porogène dans les granules par pression.

14. Procédé conforme à la revendication 13, dans lequel au moins une partie des granules sont de forme sphérique.

15. Procédé conforme à la revendication 13 ou 14, dans lequel l'agent porogène se présente sous la forme de particules, et au moins une partie de la surface de ces particules est de forme convexe.

16. Procédé conforme à l'une des revendications 13 à 15, dans lequel l'agent porogène se présente sous la forme de particules, et au moins une partie de ces particules sont sphériques.

17. Procédé conforme à l'une des revendications 13 à 16, dans lequel au moins une partie de l'agent porogène présente un diamètre valant de 10 à 250 % du diamètre des granules.

18. Procédé conforme à l'une des revendications 13 à 17, dans lequel le poids d'agent porogène utilisé se situe dans l'intervalle allant de 20 % à 80 % du poids de la quantité de granules.

19. Procédé conforme à l'une des revendications 13 à 18, dans lequel l'agent porogène comprend au moins un élément de l'ensemble constitué par les suivants :
glace, sel, polyéthylène, silicium, polystyrène, et cellulose.

20. Procédé conforme à l'une des revendications 13 à 19, dans lequel l'agent porogène comprend de la cellulose.

21. Procédé conforme à l'une des revendications 13 à 20, dans lequel l'étape d'élimination de l'agent porogène comprend une opération de brûlage, de tamisage, de dissolution, de fusion, ou de vaporisation.

22. Procédé conforme à l'une des revendications 13 à 21, dans lequel l'étape d'élimination de l'agent porogène comprend le fait de chasser l'agent porogène par brûlage.
